# EUROPEAN PATENT SPECIFICATION

(11) **EP 3 211 092 B1**
(45) Date of publication and mention of the grant of the patent: **27.03.2019**
(21) Application number: 16157229.2
(22) Date of filing: 24.02.2016
(51) Int. Cl.: C12Q 1/6869, C12Q 1/6827

(54) **SINGLE NUCLEOTIDE DETECTION METHOD**
EINZELNUKLEOTIDDETEKTIONSVERFAHREN
PROCÉDÉ DE DÉTECTION D'UN NUCLÉOTIDE SIMPLE

(43) Date of publication of application: 30.08.2017
(73) Proprietor: Base4 Innovation Ltd, Cambridge, Cambridgeshire CB3 0FA (GB)
(72) Inventor: BALMFORTH, Barnaby, Cambridge, Cambridgeshire CB3 0FA (GB)
(74) Representative: Briscoe, Paul Brian

(56) References cited:
- WO-A1-2014/167324
- WO-A1-2015/121675
- WO-A1-2016/012789

## Description

This invention relates to a method for detecting and characterising single nucleotides. It is especially suitable for use in the sequencing of DNA or RNA and detecting the location of methylated and un-methylated nucleotide bases in such sequences.

Next generation sequencing of genetic material is already making a significant impact on the biological sciences in general and medicine in particular as the unit cost of sequencing falls in line with the coming to market of faster and faster sequencing machines.

In our previous applications WO 2014/053853, WO 2014/053854, WO2014/167324, WO2014/167324 and WO2014/111723 we have described a new sequencing method which involves progressive digestion of a polynucleotide analyte to generate an ordered stream of single nucleotides, preferably a stream of single deoxyribonucleoside triphosphates, each of which can be captured one-by-one into corresponding droplets in a microdroplet stream. Thereafter, each droplet can be chemically and/or enzymatically manipulated to reveal the particular single nucleotide it originally contained. In one embodiment, these chemical and/or enzymatic manipulations comprise a method involving the use of one or more two-component oligonucleotide probe types each of which is adapted to be able to selectively capture one of the single nucleotide types from which the analyte is constituted. Typically, in each of such probe types, one of the two oligonucleotide components comprises characteristic fluorophores and in the probe's unused state the ability of these fluorophores to fluoresce remains extinguished by virtue of the presence of quenchers located close-by or by self-quenching. In use, when the probe has captured its corresponding single nucleotide, it is rendered susceptible to subsequent exonucleolysis thereby liberating the fluorophores from the quenchers and/or each other enabling them to fluoresce freely. By this means, the original single nucleotide present in each droplet can be identified indirectly by spectroscopic means.

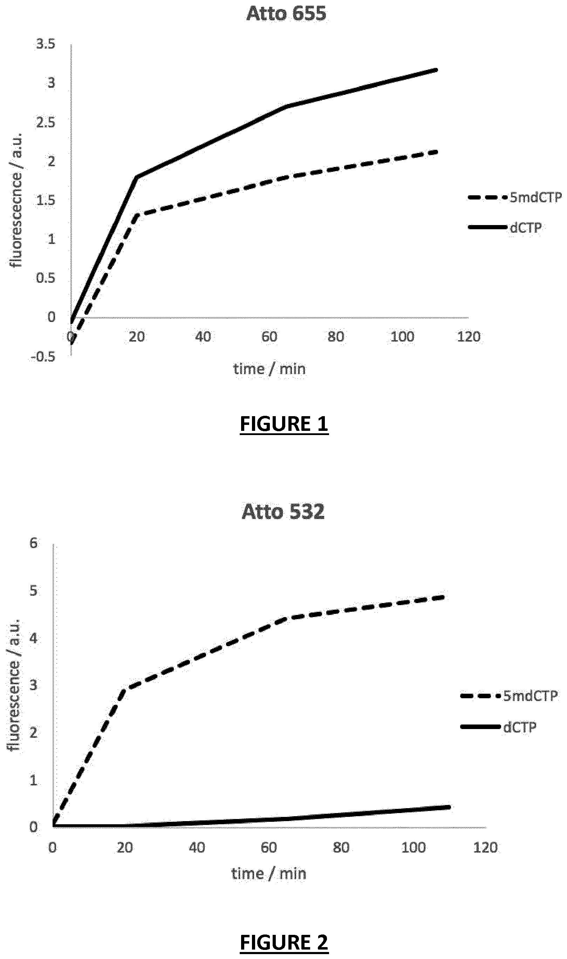

Fan et al in Nature Reviews Genetics 7(8) 632-644 (2006) provide a general review of the development of methods and platforms that have enabled highly parallel genomic assays for genotyping, copy-number measurements, sequencing and detecting loss of heterozygosity, allele-specific expression and methylation. Figure 2a of this review schematically shows the use of a circularizable probe with 3' and 5' ends that anneal upstream and downstream of a site of single nucleotide polymorphism (SNP) on an analyte thereby leaving a gap which is subsequently filled with a nucleotide which is the complement of the SNP to form a complete circular probe which may then be amplified after release. However unlike our method, the nucleotide which is captured during the filling process is not obtained directly from the analyte itself.

WO03080861 discloses a process wherein a nucleic acid analyte is subjected to progressive pyrophosphorolysis in the presence of a nucleotide-specific reactive label which attaches directly to the nucleotide as it is released. Not only is this quite different from the method we employ but in practice the fluorescence signal measured when the labelled nucleotides are subsequently interrogated would likely be too weak to enable reliable identification above the associated background noise.

Finally, WO9418218 teaches a DNA sequencing method in which the analyte is subjected to progressive exonucleolysis to generate a stream of single nucleotide diphosphates or monophosphates which are then incorporated into a fluorescence-enhancing matrix before being detected. Not only is this a completely different approach to the one we describe but we again observe that any signal generated would likely be too weak to be reliably detected and identified.

In our application WO 2016/012789 we disclose a new version of the sequencing method described in our above-mentioned patent applications involving an improved probe system. In this method, a first single-stranded oligonucleotide bearing e.g. fluorophores in an undetectable state is caused to undergo reaction in the presence of a polymerase and ligase with the nucleotide to be captured and second and third single-stranded oligonucleotides to produce a double-stranded used probe in which only the strand comprising the first oligonucleotide is susceptible to exonucleolysis. This enables the other strand produced during the capture to participate in a cycle of first oligonucleotide annealing and exonucleolysis steps so that the signal from the liberated fluorophores (now in a detectable state) grows rapidly with each cycle and becomes much easier to detect. In one embodiment, the 3' end(s) of the second and/or third oligonucleotides are rendered resistant to exonucleolysis by chemical modification or preferably linking the relevant ends of each so that when the nucleotide is captured the strand of the used probe in which these oligonucleotides are located is rendered closed-loop.

In our application WO 2015/121675 a method is disclosed for determining whether a given single nucleotide is methylated or not methylated, the method characterised by the steps of (a) contacting the single nucleotide with one or more hybridisation probe types each of which in its unused form comprises (1) a first oligonucleotide to which is attached one or more first detectable elements in an essentially undetectable state and which further comprises (i) double- and single-stranded regions and (ii) a region resistant to exonucleolytic degradation attached to the end of the double-stranded region adjacent the single- stranded region and (2) a second single-stranded oligonucleotide to which is attached one or more second detectable elements also in an essentially undetectable state and which is adapted to be at least in part the nucleotide sequence compliment of the single-stranded region of the first oligonucleotide; (b) for the relevant probe type causing (i) the single nucleotide to bind to the region resistant to exonucleolytic degradation and the single-stranded region and (ii) the second oligonucleotide to bind to the single nucleotide and the single-stranded nucleotide region to create a substantially double-stranded used probe; either (c1) treating the used probe with a methylation-dependent restriction endonuclease under conditions whereby the used probe is cleaved adjacent the region resistant to exonucleolytic degradation into two double-stranded oligonucleotide products if the single nucleotide is methylated or (c2) treating the used probe with a methylation-sensitive restriction endonuclease under conditions whereby the used probe is cleaved adjacent the region resistant to exonucleolytic degradation into two double-stranded oligonucleotide products if the single nucleotide is not methylated; and thereafter either (d1) treating the product of step (c1) with an exonuclease or a polymerase exhibiting exonuclease activity to liberate either only first or both first and second detectable elements in a detectable state if the single nucleotide is methylated, or only the second detectable elements if not or (d2) treating the product of step (c2) with an exonuclease or a polymerase exhibiting exonuclease activity to liberate either only first or both first and second detectable elements in a detectable state if the single nucleotide is not methylated, or only the second detectable element if the single nucleotide is methylated.

We have now developed an improved version of the probe system and method described in this patent application which makes it possible to distinguish between nucleotides in which the associated nucleobases are modified or unmodified; for example methylated or un-methylated. This makes the method especially suitable for example in detecting the location of methylated cytosine or adenine nucleobases in sequences. This is extremely beneficial as in some instances the locations of such methylation are known to correlate with genetic conditions and diseases such as cancer.

Thus according to a first aspect of the present invention there is provided a method of sequencing a nucleic acid characterised by the steps of (1) generating a stream of single nucleotides by progressive pyrophosphorolysis of the nucleic acid; (2) producing at least one substantially double-stranded oligonucleotide used probe by reacting, in the presence of a polymerase and a ligase, one of the single nucleotides with a corresponding probe system comprising (a) a first single-stranded oligonucleotide including first and second regions respectively labelled with different characteristic detectable element types in an undetectable state located respectively on the X' and Y' end sides of a third region comprising a restriction enzyme recognition site element including a capture site complementary to the single nucleotide and an exonuclease-blocking site on the X' side thereof (wherein either X' is 3' and Y' is 5' or X' is 5' and Y' is 3') and (b) second and third single-stranded oligonucleotides capable of hybridising to complementary regions on the first oligonucleotide flanking the capture site; (2a) either (i) treating the used probe with modification-dependent restriction endonuclease to cut only the first oligonucleotide strand at the recognition site if and only if the single nucleotide captured comprises a nucleobase which is modified or (ii) treating the used probe with a conventional or nicking modification-sensitive restriction endonuclease to cut the first oligonucleotide strand at the recognition site if and only if the single nucleotide captured comprises a nucleobase which is unmodified; (3) digesting the first oligonucleotide strand of the used probe with an enzyme having double-stranded exonucleolytic activity in the X'-Y' direction corresponding to the first oligonucleotide to yield detectable elements derived from either the first region, the second region, or the first and second regions in a detectable state and a single-stranded fourth oligonucleotide which is at least in part the sequence complement of the first oligonucleotide; (4) reacting the fourth oligonucleotide with another first oligonucleotide to produce a substantially double-stranded oligonucleotide product corresponding to the used probe; (5) repeating steps (2a), (3) and (4) in a cycle and (6) detecting the detectable elements released in each iteration of step (3) wherein if the restriction endonuclease employed is not of the nicking type the second or third oligonucleotide includes an endonucleolysis-directing linkage located at or close to its X' or Y' end respectively, so that this linkage comprises part of a restriction enzyme recognition site created in the used probe such that only the first oligonucleotide strand of the used probe is capable of being cleaved by said restriction endonuclease.

Step (1) of the method of the present invention comprises generating a stream of single nucleotides (here single nucleoside triphosphates) from a nucleic acid analyte by pyrophosphorolysis. The analyte employed in this step is suitably a double-stranded polynucleotide the length of which can in principle be unlimited including up to the many millions of nucleotide bases found in a human genome fragment. Typically however the polynucleotide will be at least 50, preferably at least 150 nucleotide pairs long; suitably it will be great than 500, greater than 1000 and in many cases thousands of nucleotide pairs long. The analyte itself is suitably RNA or DNA of natural origin (e.g. derived from a plant, animal, bacterium or a virus) although the method can also be used to sequence synthetically produced RNA or DNA or other nucleic acids made up wholly or in part of nucleobases that are not commonly encountered in nature; i.e. nucleobases other than adenine, thymine, guanine, cytosine and uracil. Examples of such nucleobases include 4-acetylcytidine, 5-(carboxyhydroxylmethyl)uridine, 2-O-methylcytidine, 5-carboxymethylaminomethyl-2-thiouridine, 5-carboxymethylamino-methyluridine, dihydrouridine, 2-O-methylpseudouridine, 2-O-methylguanosine, inosine, N6-isopentyladenosine, 1-methyladenosine, 1-methylpseudouridine, 1-methylguanosine, 1-methylinosine, 2,2-dimethylguanosine, 2-methyladenosine, 2-methylguanosine, 3-methylcytidine, 5-methylcytidine, N6-methyladenosine, 7-methylguanosine, 5-methylaminomethyluridine, 5-methoxyaminomethyl-2-thiouridine, 5-methoxyuridine, 5-methoxycarbonylmethyl-2-thiouridine, 5-methoxycarbonylmethyluridine, 2-methylthio-N6-isopentenyladenosine, uridine-5-oxyacetic acid-methylester, uridine-5-oxyacetic acid, wybutoxosine, wybutosine, pseudouridine, queuosine, 2-thiocytidine, 5-methyl-2-thiouridine, 2-thiouridine, 4-thiouridine, 5-methyluridine, 2-O-methyl-5-methyluridine and 2-O-methyluridine. In the case of DNA the single nucleotides generated are deoxyribonucleoside triphosphates whilst in the case of RNA they are ribonucleoside triphosphates.

In one embodiment of the invention, step (1) comprises a first sub-step of attaching the analyte to a substrate. Typically, the substrate comprises a microfluidic surface, a micro-bead or a permeable membrane; for example one made out of glass or a non-degradable polymer. Preferably, the substrate further comprises a surface adapted to receive the analyte. There are many ways in which the analyte can be attached to such surfaces any of which can in principle be used in this sub-step. For example, one method involves priming a glass surface with a functionalised silane such as an epoxysilane, an aminohydrocarbylsilane or a mercaptosilane. The reactive sites so generated can then be treated with a derivative of the analyte which has been modified to include a terminal amine, succinyl or thiol group.

In one embodiment of step (1), the analyte is pyrophosphorolysed progressively to generate a stream of single nucleotides the order of which corresponds to that of the sequence of the analyte. Pyrophosphorolysis may be carried out at a temperature in the range 20 to 90°C in the presence of a reaction medium comprising a polymerase. Preferably it is carried out under conditions of continuous flow so that the single nucleotides are continually removed from the reaction zone as they are liberated. Most preferably, the pyrophosphorolysis is carried out by causing an aqueous buffered medium containing the enzyme and the other typical additives to continuously flow over the surface to which the analyte is bound.

In one embodiment, the enzyme used is one which can cause progressive 3'-5' pyrophosphorolytic degradation of the analyte to yield a stream of nucleotides with high fidelity and at a reasonable reaction rate. Preferably this degradation rate is as fast as possible and in one embodiment is in the range 1 to 50 nucleotides per second. Further information about the pyrophosphorolysis reaction as applied to the degradation of polynucleotides can be found for example in J. Biol. Chem. 244 (1969) pp. 3019-3028. Suitably, the pyrophosphorolysis is carried out in the presence of a medium which further comprises pyrophosphate anion and magnesium cations; preferably in millimolar concentrations.

In step (2) of the method of the present invention at least one single nucleotide in the stream, preferably each single nucleotide in an ordered stream, is reacted, in the presence of a polymerase and a ligase, with a probe system to generate a substantially double-stranded used probe. In one embodiment, before this step is carried out the product of step (1) is treated with a pyrophosphatase to hydrolyse any residual pyrophosphate to phosphate anion.

The polymerase used in this step is in one embodiment selected from the group consisting of those which show essentially neither exo- nor endonuclease activity under the reaction conditions. Examples of polymerases which can be advantageously used include, but are not limited to, the prokaryotic pol 1 enzymes or enzyme derivatives obtained from bacteria such as *Escherichia coli* (e.g. Klenow fragment polymerase), *Thermus aquaticus* (e.g. *Taq* Pol), *Bacillus stearothermophilus, Bacillus caldovelox* and *Bacillus caldotenax.* Any suitable ligase can be used in this step.

The probe system employed in step (2) is comprised of three components; (a) a first single-stranded oligonucleotide including first and second regions respectively labelled with different characteristic detectable element types in an undetectable state located respectively on the X' and Y' sides of a third region comprising a restriction enzyme recognition site element including a capture site complementary to the single nucleotide and an exonuclease-blocking site on the X' side thereof, wherein either X' is 3' and Y' is 5' or X' is 5' and Y' is 3', and (b) second and third unlabelled single-stranded oligonucleotides capable of hybridising to complementary regions on the first oligonucleotide flanking the capture site. In one embodiment, where the restriction endonuclease is not a nicking endonuclease, the second and/or third oligonucleotide includes an endonucleolysis-directing linkage located at or close to their X' or Y' end respectively so that ultimately this linkage comprises part of a restriction enzyme recognition site created in the used probe. In one embodiment this endonucleolysis-directing linkage is a phosphorothioate linkage. In another, it is one selected from the group consisting of phosphoramidite linkages such as C3 spacer, Spacer 9, Spacer 12, Spacer 18, d-Spacer (abasic furan type) or other like spacers or modifications used between nucleotides in oligonucleotide synthesis.

In one embodiment, the second and third oligonucleotides are discrete entities whilst in another they are linked to each other by means of a linker region. In another, the linker region links the Y' end of the second oligonucleotide and the X' end of the third oligonucleotide together where here and hereinafter the terms X' and Y' are used with respect to the direction in which the exonucleolysis in step (3) occurs. This linker region can in principle be any divalent group but is conveniently another single- or double-stranded oligonucleotide fragment. In one embodiment, the linker region is unable to hybridise substantially to the first oligonucleotide.

The first oligonucleotide further includes first and second regions bearing different detectable elements in an undetectable state on the X' and Y' end sides of a third region located therebetween which includes the capture site mentioned above. This capture site comprises a single nucleotide whose nucleobase is complementary to the one borne by the nucleotide to be detected, making the probe system highly selective for that particular nucleotide. Thus, for example, if the analyte is derived from DNA and the first, second and third oligonucleotides are deoxyribonucleotides, the capture site will be highly selective for deoxyadenosine triphosphate if the capture site nucleotide bears a thymine base. In one useful embodiment of the invention therefore step (2) may be carried out in the presence of a plurality of probe system types; for example one, two, three or four probe system types each of which comprises a first oligonucleotide having (a) a different capture site nucleotide characteristic of the various different nucleobases sought and (b) different associated detectable elements. In one embodiment all the first oligonucleotides are of the type described herein. In another a mixture of at least one of this type with one or more of the first oligonucleotides described in WO 2016/012789 is employed.

The capture site itself forms part of a restriction site element which enables a restriction enzyme recognition site to be created when the used probe is formed. Typically the restriction site element is between 4 and 8 nucleotides long, but in principle it may be of any length.

The third region also includes an exonuclease-blocking site designed to halt X'-Y' exonucleolytic digestion of the first oligonucleotide strand of the used probe in step (3) before the recognition site is reached. By this means, the detectable elements associated with both first and second regions can only be released in detectable form if the first oligonucleotide strand of the used probe is cleaved at the recognition site. This exonuclease-blocking element is therefore suitably located in the backbone of the third region on the X' end side of the restriction site element. It is suitably an element selected from a phosphorothioate linkage, a G-Quadruplex, a boronated nucleotide, an inverted dT or ddT, a C3 spacer, a phosphate group, or any of the various exonuclease-inhibiting linkers or spacers commonly available as internal oligonucleotide modifications.

Typically, the first oligonucleotide is up to 150 nucleotides long, preferably between 20 and 100 nucleotides. In one embodiment, the second oligonucleotide is longer than the complementary X' side flanking region of the first oligonucleotide by up to 10 preferably from 1 to 5 nucleotides. In another, there is a single nucleotide mismatch between the 3' end of the first oligonucleotide and the nucleotide opposite it on the second or third oligonucleotide to prevent the nucleoside triphosphate being captured by the polymerase at this point. In yet another embodiment, the X' end of the third oligonucleotide includes an element resistant to exonucleolytic degradation to ensure that the fourth oligonucleotide produced in step (3) is not itself subsequently digested. This can be achieved for example by way of incorporating one or more phosphorothioate linkages, a G-Quadruplex, a boronated nucleotide, an inverted dT or ddT, a C3 spacer, a phosphate group or any of the various exonuclease-inhibiting linkers and spacers commonly available, at or near that particular end.

It is a feature of the first oligonucleotide that it is labelled with first and second detectable element regions each of which is labelled, preferably multiply labelled, with its own unique and characteristic type of detectable element(s) and that these detectable elements are substantially undetectable when the probe system is in an unused state. Suitably these detectable elements are ones adapted to be detected after an optical event has taken place. In one preferred embodiment, the detectable elements comprise fluorophores and each unused first oligonucleotide is essentially non-fluorescing at those wavelengths where the fluorophores are designed to be detected. Thus, although a fluorophore may exhibit general, low-level background fluorescence across a wide part of the electromagnetic spectrum, there will typically be one or a small number of specific wavelengths or wavelength envelopes where the intensity of the fluorescence is at a maximum. It is at one or more of these maxima where the fluorophore is characteristically detected that essentially no fluorescence should occur. In the context of this patent, by the term 'essentially non-fluorescing' or equivalent wording is meant that the intensity of fluorescence of the total number of fluorophores attached to the first oligonucleotide at the relevant characteristic wavelength or wavelength envelope is less than 25%; preferably less than 10%; more preferably less than 1% and most preferably less than 0.1% of the corresponding intensity of fluorescence of an equivalent number of free fluorophores.

If fluorophores are employed in a given first and/or second region, then in principle, any method can be used to ensure that in the first oligonucleotide's unused state they are essentially non-fluorescing. One approach is to additionally attach quenchers in close proximity to them. Another is based on the observation that when multiple fluorophores are located in close proximity to each other they tend to quench each other sufficiently well that the criterion described in the previous paragraph can be achieved without the need for quenchers. In this context of this patent, what constitutes 'close proximity' between fluorophores or between fluorophores and quenchers will depend on the particular fluorophores and quenchers used and possibly the structural characteristics of the first oligonucleotide. Consequently, it is intended that this term should be construed with reference to the required outcome rather than any particular structural arrangement of the various elements. However, and for the purposes of providing exemplification only, it is pointed out that when adjacent fluorophores or adjacent fluorophores and quenchers are separated by a distance corresponding to the characteristic Forster distance (typically less than 5nm) sufficient quenching will generally be achieved.

Suitably each first and second region is labelled with at least 1, preferably up to 20 fluorophores. To obtain maximum advantage, it is preferred that each region is labelled with at least 2 preferably at least 3 fluorophores. Consequently, ranges constructed from any permutation of these maximum and minimum numbers are specifically envisaged herein. If quenchers are employed, it is likewise preferred that each region is labelled with up to 20, preferably up to 10 and most preferably up to 5 of the same.

As regards the fluorophores themselves, they can in principle be chosen from any of those conventionally used in the art including but not limited to xanthene moieties e.g. fluorescein, rhodamine and their derivatives such as fluorescein isothiocyanate, rhodamine B and the like; coumarin moieties (e.g. hydroxyl-, methyl- and aminocoumarin) and cyanine moieties such as Cy2, Cy3, Cy5 and Cy7. Specific examples include fluorophores derived from the following commonly used dyes: Alexa dyes, cyanine dyes, Atto Tec dyes, and rhodamine dyes. Examples also include: Atto 633 (ATTO-TEC GmbH), Texas Red™, Atto 740 (ATTO-TEC GmbH), Rose Bengal, Alexa Fluor™ 750 C₅-maleimide (Invitrogen), Alexa Fluor™ 532 C₂-maleimide (Invitrogen) and Rhodamine Red C₂-maleimide and Rhodamine Green as well as phosphoramadite dyes such as Quasar 570. Alternatively, a quantum dot or a near infra-red dye such as those supplied by LI-COR Biosciences can be employed. The fluorophore is typically attached to the first oligonucleotide via a nucleotide base using chemical methods known in the art.

Suitable quenchers include those which work by a Forster resonance energy transfer (FRET) mechanism. Examples of commercially available quenchers which can be used in association with the above mentioned-fluorophores include but are not limited to DDQ-1, Dabcyl, Eclipse, Iowa Black FQ and RQ, IR Dye-QC1, BHQ-0, BHQ-1, -2 and -3 and QSY-7 and -21.

In one embodiment the second and third oligonucleotides are not labelled with detectable elements.

Step (2) is suitably carried out by contacting each single nucleotide in the stream with one or more probe systems as described above at a temperature in the range 20 to 80°C.

The product of step (2) of the method of the invention is, as mentioned above, a substantially double-stranded used probe whose constituent strands are respectively the first oligonucleotide and a complementary fourth oligonucleotide which when read in its Y'-X' direction is comprised of the second oligonucleotide, the captured nucleotide and finally the third oligonucleotide. Thus, if the second and third oligonucleotides have previously been joined together by a linker region it will be readily apparent that the fourth oligonucleotide will comprise a closed loop strand that is highly resistant to exonucleolysis.

In step (2a) the used probe is treated with a modification-dependent or modification sensitive restriction endonuclease to enable the method to detect the presence or absence of a nucleobase modification (e.g. an alkyl, amino, nitro, halide, sulphur or like modification) in the single nucleotide. By this means the method can be used, for example, to detect the presence or absence of methylated nucleobases although this should not be construed as limiting. In this particular application, either a methylation-dependent or a methylation-sensitive restriction endonuclease, preferably at a temperature in the range up to 70°C, is used to cleave the first oligonucleotide strand at the recognition site if and only if the single nucleotide captured comprises a methylated or non-methylation nucleobase (as the case may be). For example, the methylation-sensitive endonucleases Mspl and Hpall cleave double-stranded oligonucleotides only at a recognition site having the sequence 5'--CC*GG--3' where C* is non-methylated cytosine and the Dnpl endonuclease cleaves only at a recognition site having the sequence 5'--GA*TC--3' where A* is methylated adenine. It is an important feature of this step that only the first oligonucleotide strand of the used probe is capable of being cleaved by the endonuclease either by virtue of the restriction enzyme being a nicking endonuclease or by virtue of the presence of the endonucleolysis-directing linkage derived from the second or third oligonucleotide in the other strand. As a consequence, if the endonuclease employed is methylation-dependent and the single nucleotide captured by the capture site is methylated the restriction endonuclease will cleave the first oligonucleotide strand of the used probe in two, making both first and second regions susceptible to digestion in step (3) whilst if the single nucleotide captured is un-methylated the restriction enzyme will have no effect and digestion of the first oligonucleotide strand will continue in a X'-Y' direction until stopped by the exonuclease-blocking site. Thus, only the detectable elements associated with the first region will be liberated in a detectable form. Conversely, if the endonuclease employed is methylation-sensitive and the single nucleotide captured is non-methylated the restriction endonuclease will cleave the first oligonucleotide strand of the used probe in two, making both first and second regions susceptible to digestion in step (3) whilst if the single nucleotide captured is methylated the restriction enzyme will have no effect and digestion of the first nucleotide strand will continue in a X'-Y' direction until stopped by the exonuclease-blocking site. Thus, only the detectable elements associated with the first region will be liberated in a detectable form. This means that in step (6) the observer will detect both types of detectable element if the single nucleotide captured was methylated and only the second type if it was un-methylated when using a methylation-dependent restriction enzyme, with the opposite being true when using a methylation-sensitive restriction enzyme. In another embodiment, cleavage at the restriction site allows the oligonucleotide fragment comprising the first region to melt, resulting in only the second region being digested, whilst lack of cleavage again results in the digestion of only the first region.

In step (3) the product of step (2a) is treated with an enzyme at a temperature in the range 30 to 100°C. In this step the strand or parts of the strand of the used probe derived from the first oligonucleotide are digested in the X'-Y' direction into their constituent nucleotides (deoxyribonucleoside monophosphates or ribonucleoside monophosphates as the case may be) in the process separating the detectable elements from each other and causing them to become unquenched and therefore detectable. Thus if the detectable elements are fluorophores which have been quenched into an undetectable state in the first oligonucleotide, step (3) will liberate the fluorophores from each other and any quenchers thereby causing them to fluoresce. As the digestion process occurs the observer therefore sees rapid growth in the fluorescence signal or signals as a cascade of fluorophores is generated. The characteristics of this fluorescence then indirectly reflects the nature of the single nucleotide originally captured by the relevant probe system.

Enzymes which can be used in step (3) comprise exonucleases or polymerases which exhibit 3'-5' or 5'-3' exonucleolytic activity. The 3'-5' class of enzyme includes Q5, Q5 Hot Start, Phusion, Phusion HS, Phusion II, Phusion II HS, Dnase I (RNase-free), Exonuclease I or III (ex *E.coli*), Exonuclease T, Exonuclease V (RecBCD), Lambda Exonuclease, Micrococcal Nuclease, Mung Bean Nuclease, Nuclease BAL-31, RecJ_{f}, T5 Exonuclease and T7 Exonuclease. Step (3) is preferably carried out at a temperature in the range 60-90°C.

In one embodiment, at the end of step (3) and before step (4) the reaction mixture is preferably heated to a temperature in the range 80-100°C to remove from the fourth oligonucleotide any attached residual oligonucleotide fragments left over from the digestion.

In step (4) the fourth oligonucleotide, now present in single-stranded form, is caused to hybridise to another first oligonucleotide molecule thereby producing a new substantially double-stranded oligonucleotide product corresponding to, i.e. one having the same chemical and physical structure as the used probe. This product is then digested in a repeat of steps (2a) and (3) thereby releasing further detectable elements in a detectable state and again regenerating the fourth oligonucleotide. Thereafter, according to step (5), steps (2a), (3) and (4) are allowed to continue in a cyclic way causing further enhancement in the signal from the free detectable elements, e.g. the fluorescence signal, in principle until substantially all of the first oligonucleotide has been consumed. As a consequence the observer sees a much greater enhancement of the fluorescence signal than has been previously obtained.

Thereafter, and in step (6), the detectable elements liberated in the various iterations of step (3) are detected and the nature of the nucleobase attached to the original single nucleotide determined. By carrying out the method of the invention systematically for all the single nucleotides in the stream generated in step (1), data characteristic of the sequence of original nucleic acid analyte can therefore be generated and methylation sites located. Methods of doing this detection are well-known in the art; for example fluorescence may be detected using a photodetector or an equivalent device tuned to the characteristic fluorescence wavelength(s) or wavelength envelope(s) of the various fluorophore types. This in turn causes the photodetector to generate a characteristic electrical signal which can be processed and analysed in a computer using known algorithms. In one embodiment, a period of time is allowed to elapse between steps (5) and (6) to ensure that the number of detectable elements in a detectable state has grown to a maximum.

In one particularly preferred embodiment, the method of the present invention is carried out wholly or partially in a stream of microdroplets, at least some of which contain a single nucleotide; suitably an ordered stream. Such a method may begin, for example, by inserting the nucleotides generated in step (1) one-by-one into a corresponding stream of aqueous microdroplets maintained in an immiscible carrier solvent such as a hydrocarbon or silicone oil to help preserve the ordering. Advantageously, this can be achieved by directly creating the microdroplets downstream of the pyrophosphorolysis reaction zone; for example by causing the reaction medium to emerge from a microdroplet head of suitable dimensions into a flowing stream of the solvent. Alternatively, small aliquots of the reaction medium from step (1) can be regularly and sequentially injected into a stream of pre-existing aqueous microdroplets suspended in the solvent. If this latter approach is adopted, each microdroplet may already contain the various components of the probe system(s) together with the enzymes and any other reagents (e.g. buffer) required to effect steps (2) to (5). In yet another approach, the microdroplets created in the former embodiment can be caused to coalesce subsequently with a stream of such pre-existing microdroplets to achieve a similar outcome. In these microdroplet methods, step (6) then preferably involves interrogating each microdroplet to identify the detectable elements liberated and hence the nature of the nucleoside triphosphate it originally contained. However in a most preferred embodiment of all the reaction medium from step (1) is printed as a series of microdroplets onto a moveable surface covered with the carrier solvent at droplet-receiving locations on the surface optionally comprised of wells and the like. Thereafter, the other components mentioned above may be introduced sequentially into each location in the form of further aqueous microdroplets which undergo coalescence with the original reaction medium. If so desired, these further aqueous microdroplets may be introduced only after an incubation period has elapsed. If this approach is adopted then step (6) involves the interrogation of each droplet-receiving location in turn.

In any of these approaches, to avoid the risk that a given microdroplet contains more than one nucleotide it is preferred to release each nucleotide in step (1) at a rate such that each filled microdroplet is separated on average by from 1 to 20 preferably 2 to 10 empty ones. Suitably the microdroplets employed have a finite diameter of less than 100 microns, preferably less than 50 microns, more preferably less than 20 microns and even more preferably less than 15 microns. Most preferably of all their diameters are in the range 2 to 20 microns. In one embodiment, the microdroplet generation rate through the whole system is in the range 50 to 3000 microdroplets per second preferably 100 to 2000.

According to a second aspect of the invention there is provided a multi-component biological probe system characterised by comprising (a) a first single-stranded oligonucleotide labelled with first and second regions of different detectable element types in an undetectable state located respectively on the X' and Y' end sides of a third region comprising a single nucleotide capture site complementary to one of the nucleobases of DNA or RNA having (1) an associated restriction enzyme recognition site element and (2) an exonuclease-blocking site on its X' side and (b) second and third unlabelled single-stranded oligonucleotides capable of hybridising respectively to the complementary X' and Y' side regions on the first oligonucleotide flanking the capture wherein either X' is 3' and Y' is 5' or X is 5' and Y is 3'.

In a first embodiment the third oligonucleotide includes an endonucleolysis-directing linkage located at or close to its Y' end. In another the second oligonucleotide has a similar linkage located at or close to its X' end. In one embodiment this linkage is a phosphorothioate linkage. In another, it is one selected from the group consisting of phosphoramidite linkages such as C3 spacer, Spacer 9, Spacer 12, Spacer 18, d-Spacer (abasic furan type) or other like spacers or modifications used between nucleotides in oligonucleotide synthesis. Preferably, the Y' end of the second oligonucleotide and X' end of the third oligonucleotide are connected by a linker region suitably a single- or double-stranded oligonucleotide region. In another embodiment the detectable elements in the first and second regions are fluorophores of the type mentioned above optionally in the presence of quenchers. In yet another embodiment the second oligonucleotide is longer than the X' flanking region of the first oligonucleotide. Preferably, the nucleotide at the 3' end of the first oligonucleotide is a mismatch with the corresponding nucleotide in the second or third oligonucleotide.

When the second or third oligonucleotides are not connected by a linker region the second and/or third oligonucleotide suitably also includes an element resistant to exonucleolytic degradation at its X' end.

The method and probe systems described above can be used to advantage in a sequencing device and such devices and uses are envisaged as being within the scope of the invention.

The present invention in its various aspects will now be illustrated with reference to the following example.

### Example 1 - Preparation and use of a probe system

A single-stranded first oligonucleotide 1 was prepared, having the following nucleotide sequence:
5'-ACATCACGACTATCTAYYZCAGGACGG/mC/CCGTTAT/C3/CAXXQAACCGCACGA*-3'
wherein A, C, G, and T represent nucleotides bearing the relevant characteristic nucleotide base of DNA; X represents a deoxythymidine nucleotide (T) labelled with Atto 655 dye, Q represents a deoxythymidine nucleotide labelled with a BHQ-2 quencher, Y represents a deoxythymidine nucleotide labelled with Atto 532 dye, Z represents a deoxythymidine nucleotide labelled with a BHQ-1 quencher, /C3/ represents a C3-spacer modification, /mC/represents a 5-methylcytosine nucleotide and * represents a hexanediol modification. It further comprises a capture region (G nucleotide) selective for capturing deoxycytosine triphosphate nucleotides (dCTPs) in a mixture of deoxynucleotide triphosphates (dNTPs).

Two further single-stranded oligonucleotides 2 and 3 were also prepared, each having regions complementary to regions of oligonucleotide 1, with a single base gap formed at the capture region of oligonucleotide 1 when both oligonucleotides 2 and 3 are annealed thereto. Oligonucleotide 2 had the following nucleotide sequence:
5'-GTGATGTTAACGTGCGGTTAAATGATAACG**GG-3'
wherein ** represents a phosphorothioate linkage. Oligonucleotide 3 had the following sequence:
5'-PCGTCCTGAAATAGATAGTCGTGATGTCTAGCATGACATA/IdT/-3'
wherein P represents a 5' phosphate group and /IdT/ represents a 3' inverted dT base.

A reaction mixture comprising the probe system was then prepared. It had a composition corresponding to that derived from the following formulation:
20nM oligonucleotide 1
10nM oligonucleotide 2
50nM oligonucleotide 3
10nM dCTP, 5mdCTP or equivalent volume of water
1x buffer pH 7.6
1.4 reactions/mL Pfu Ultra Fusion II Hot Start polymerase
57U/mL Bst Large Fragment polymerase
71U/mL E. coli ligase
3.6U/mL AOXI
14U/mLThermostable Inorganic Pyrophosphatase
wherein 5x buffer comprised the following mixture:
60uL Trizma hydrochloride, 1M, pH 7.6
33.3uL KCl, 3M
25uL aqueous MgCl₂, 1M
20uL Nicotinamide adenine dinucleotide, 100 uM
2.5uL Dithiothreitol, 1M
50uL Triton X-100 surfactant (10%)
Water to 1mL

Capture of the dCTPs or 5mdCTPs and ligation of oligonucleotide 2 to oligonucleotide 3 was then carried out by incubating the mixture at 37°C for 10 minutes after which the temperature was increased to 60°C for a further 20 minutes. The temperature was then increased to 95°C for 30 seconds before finally being decreased to 70°C for 90 minutes. The reaction mixture was then illuminated using the 633nm line of a Helium-Neon laser and the 532nm line of a diode laser and the resulting characteristic fluorescence of the Atto 655 and Atto 532 dyes detected using a camera.

The growth over time in intensity of fluorescence over background in the presence of the dNTP component of the reaction was monitored and the results shown graphically in figures 1 and 2. From these it can be seen that the probe system efficiently captures both dCTPs and 5mdCTPs. In the presence of dCTP a fluorescence signal is observed only in the Atto 655 channel while in the presence of 5mdCTP a signal is observed in both the Atto 655 and Atto 532 channels. In a comparative experiment where no dNTPs were present in the reaction mixture neither of the Atto 655 and Atto 532 dyes on oligonucleotide 1 exhibited fluorescence to any significant extent.

## Claims

1. A method of sequencing a nucleic acid **characterised by** the steps of (1) generating a stream of single nucleotides by progressive pyrophosphorolysis of the nucleic acid; (2) producing at least one substantially double-stranded oligonucleotide used probe by reacting, in the presence of a polymerase and a ligase, one of the single nucleotides with a corresponding probe system comprising (a) a first single-stranded oligonucleotide including first and second regions respectively labelled with different characteristic detectable element types in an undetectable state located respectively on the X' and Y' sides of a third region comprising a restriction enzyme recognition site element including a capture site complementary to the single nucleotide and an exonuclease-blocking site on the X' side thereof, wherein either X' is 3' and Y' is 5' or X' is 5' and Y' is 3', and (b) second and third single-stranded oligonucleotides capable of hybridising to complementary regions on the first oligonucleotide flanking the capture site; (2a) either (i) treating the used probe with a modification-dependent restriction endonuclease to cut only the first oligonucleotide strand at the recognition site if and only if the single nucleotide captured comprises a nucleobase which is structurally modified or (ii) treating the used probe with modification-sensitive restriction endonuclease to cut only the first oligonucleotide strand at the recognition site if and only if the single nucleotide captured comprises a nucleobase which is unmodified; (3) digesting the first oligonucleotide strand of the used probe with an enzyme having double-stranded exonucleolytic activity in the X'-Y' direction corresponding to the first oligonucleotide to yield detectable elements derived from either the first region, the second region, or the first and second regions in a detectable state and a single-stranded fourth oligonucleotide which is at least in part the sequence complement of the first oligonucleotide; (4) reacting the fourth oligonucleotide with another first oligonucleotide to produce a substantially double-stranded oligonucleotide product corresponding to the used probe; (5) repeating steps (2a), (3) and (4) in a cycle and (6) detecting the detectable elements released in each iteration of step (3) wherein if the restriction endonuclease employed is not of the nicking type the second or third oligonucleotide includes an endonucleolysis-directing linkage located at or close to its X' or Y' end respectively, so that this linkage comprises part of a restriction enzyme recognition site created in the used probe such that only the first oligonucleotide strand of the used probe is capable of being cleaved by said restriction endonuclease.

2. A method as claimed in claim 1 **characterised in that** the Y' end of the second oligonucleotide and the X' end of the third oligonucleotide are connected by a linker region.

3. A method as claimed in claim 2 **characterised in that** the linker region comprises an oligonucleotide region.

4. A method as claimed in claim 2 or 3 **characterised in that** the fourth oligonucleotide generated comprises a closed loop.

5. A method as claimed in any of the preceding claims **characterised in that** the second oligonucleotide (a) hybridises to the flanking region on the X' side of the capture region and (b) is longer than the region on that side.

6. A method as claimed in any of the preceding claims **characterised in that** there is at least one nucleotide base mismatch between the 3' end of the first oligonucleotide and the corresponding region of the second or third oligonucleotide.

7. A method as claimed in any of the preceding claims **characterised in that** the detectable element types are fluorophore types rendered undetectable in the first oligonucleotide by the presence of at least one quencher.

8. A method as claimed in any of the preceding claims **characterised in that** the modified single nucleoside triphosphate detected includes either a methylated cytosine or methylated adenine nucleotide base.

9. A method as claimed in any of the preceding claims **characterised in that** steps (2a) and (3) are carried out at different temperatures.

10. A method as claimed in any of the preceding claims **characterised in that** the probe system comprises a plurality of first oligonucleotide types each provided with a different capture region and characteristic first or first and second detectable element types.

11. A method as claimed in claim 10 **characterised in that** up to four different sets of oligonucleotide probe systems are employed, the first oligonucleotide of each set having a capture region selective for one of the characteristic nucleotide bases of naturally-occurring DNA or RNA and different first or pairs of first and second detectable element types.

12. A method as claimed in any of the preceding claims **characterised in that** step (1) further comprises containing each single nucleoside triphosphate in a corresponding microdroplet and that steps (2) to (6) and (2a) are carried out in each microdroplet.

13. A method as claimed in claim 12 **characterised in that** the results obtained by applying step (6) to each microdroplet are assembled into a stream of data characteristic of the sequence of the nucleic acid.

14. A multi-component biological probe system **characterised by** comprising (a) a first single-stranded oligonucleotide labelled with first and second regions of different detectable element types in an undetectable state located respectively on the X' and Y' end sides of a third region comprising a single nucleotide capture site complementary to one of the nucleobases of DNA or RNA having (1) an associated restriction enzyme recognition site and (2) an exonuclease-blocking site on its X' side and (b) second and third unlabelled single-stranded oligonucleotides capable of hybridising respectively to the complementary X' and Y' side regions on the first oligonucleotide flanking the capture site wherein either X' is 3' and Y' is 5' or X' is 5' and Y' is 3'.

15. A biological probe system as claimed in claim 14 **characterised in that** the second or third oligonucleotide includes an endonucleolysis-directing linkage located at or close to its X' or Y' end respectively, so that this linkage comprises part of a restriction enzyme recognition site created in the used probe such that only the first oligonucleotide strand of the used probe is capable of being cleaved by said restriction endonuclease.

16. A biological probe system as claimed in claim 14 or 15 **characterised in that** the Y' end of the second oligonucleotide and the X' end of the third oligonucleotide are connected by a linker region.

17. A biological probe system as claimed in claim 16 **characterised in that** the linker region comprises an oligonucleotide region.

18. A biological probe system as claimed in any of claims 14 to 17 **characterised in that** the detectable elements in the first and second regions are fluorophores optionally associated with quenchers.

19. A biological probe system as claimed in any of claims 14 to 18 **characterised in that** the second oligonucleotide is longer than the X' side flanking region of the first oligonucleotide.

20. A biological probe system as claimed in any of claims 14 to 19 **characterised in that** the nucleotide at the 3' end of the first oligonucleotide is a mismatch with the corresponding nucleotide in the second or third oligonucleotide.

21. A biological probe system as claimed in claims 14 to 20 **characterised in that** the X' end of the third oligonucleotide comprises a region resistant to exonucleolysis.

22. A biological probe system as claimed in claims 15 to 21 **characterised in that** the endonucleolysis-directing linkage is a phosphorothioate or phosphoramidite linkage.

23. A biological probe system as claimed in claims 14 to 21 **characterised by** comprising from one to four different first oligonucleotides types differing in sequence, the nucleotide base characteristic of the capture region, and the detectable elements used.

## Patentansprüche

1. Verfahren zum Sequenzieren einer Nukleinsäure, **gekennzeichnet durch** die Schritte (1) Erzeugen eines Stroms von Einzelnukleotiden durch fortschreitende Pyrophosphorolyse der Nukleinsäure; (2) Herstellen mindestens einer im Wesentlichen doppelsträngigen verwendeten Oligonukleotid-Sonde durch in Gegenwart einer Polymerase und einer Ligase erfolgendes Umsetzen eines der Einzelnukleotide mit einem entsprechenden Sondensystem, umfassend (a) ein erstes einzelsträngiges Oligonukleotid, beinhaltend erste und zweite Regionen, die jeweils mit unterschiedlichen charakteristischen detektierbaren Elementtypen in einem nicht-detektierbaren Zustand markiert sind, die sich jeweils auf der X'- und Y'-Seite einer dritten Region befinden, umfassend ein Restriktionsenzym-Erkennungsstellenelement, einschließlich einer Einfangstelle, die zu dem Einzelnukleotid komplementär ist, und einer Exonuklease-Blockierungsstelle auf der X'-Seite davon, wobei entweder X' 3' liegt und Y' 5' liegt oder X' 5' liegt und Y' 3' liegt, und (b) zweite und dritte einzelsträngige Oligonukleotide, die befähigt sind zur Hybridisierung an komplementäre Regionen auf dem ersten Oligonukleotid, welche die Einfangstelle flankieren; (2a) entweder (i) Behandeln der verwendeten Sonde mit einer modifikationsabhängigen Restriktionsendonuklease, um nur den ersten Oligonukleotidstrang an der Erkennungsstelle dann und nur dann zu schneiden, wenn das eingefangene Einzelnukleotid eine Nukleobase umfasst, die strukturell modifiziert ist, oder (ii) Behandeln der verwendeten Sonde mit modifikationsempfindlicher Restriktionsendonuklease, um nur den ersten Oligonukleotidstrang an der Erkennungsstelle dann und nur dann zu schneiden, wenn das eingefangene Einzelnukleotid eine Nukleobase umfasst, die unmodifiziert ist; (3) Verdauen des ersten Oligonukleotidstrangs der verwendeten Sonde mit einem Enzym, aufweisend doppelsträngige exonukleolytische Aktivität in der X'-Y'-Richtung, entsprechend dem ersten Oligonukleotid, unter Erhalt von detektierbaren Elementen, die abgeleitet sind entweder aus der ersten Region, der zweiten Region oder der ersten und der zweiten Region in einem detektierbaren Zustand, und einem einzelsträngigen vierten Oligonukleotid, das zumindest teilweise das Sequenzkomplement des ersten Oligonukleotids ist; (4) Umsetzen des vierten Oligonukleotids mit einem weiteren ersten Oligonukleotid zur Erzeugung eines im Wesentlichen doppelsträngigen Oligonukleotidprodukts entsprechend der verwendeten Sonde; (5) Wiederholen der Schritte (2a), (3) und (4) in einem Zyklus; und (6) Erfassen der detektierbaren Elemente, die bei jeder Iteration von Schritt (3) freigesetzt wurden, wobei, wenn die angewendete Restriktionsendonuklease nicht vom Nicking-Typ ist, das zweite oder dritte Oligonukleotid eine Endonukleolysedirigierende Verknüpfung einschließt, die sich an oder nahe seinem X'- bzw. Y'-Ende befindet, so dass diese Verknüpfung einen Teil einer Restriktionsenzym-Erkennungsstelle umfasst, die in der verwendeten Sonde erzeugt wird, so dass nur der erste Oligonukleotidstrang der verwendeten Sonde befähigt ist, durch die genannte Restriktionsendonuklease gespalten zu werden.

2. Verfahren, wie in Anspruch 1 beansprucht, **dadurch gekennzeichnet, dass** das Y'-Ende des zweiten Oligonukleotids und das X'-Ende des dritten Oligonukleotids durch eine Linkerregion verbunden sind.

3. Verfahren, wie in Anspruch 2 beansprucht, **dadurch gekennzeichnet, dass** die Linkerregion eine Oligonukleotidregion umfasst.

4. Verfahren, wie in Anspruch 2 oder 3 beansprucht, **dadurch gekennzeichnet, dass** das vierte erzeugte Oligonukleotid eine geschlossene Schleife umfasst.

5. Verfahren, wie in einem der vorhergehenden Ansprüche beansprucht, **dadurch gekennzeichnet, dass** das zweite Oligonukleotid (a) an die flankierende Region auf der X'-Seite der Einfangregion hybridisiert und (b) länger ist als die Region auf dieser Seite.

6. Verfahren, wie in einem der vorhergehenden Ansprüche beansprucht, **dadurch gekennzeichnet, dass** mindestens eine Nukleotidbasen-Fehlpaarung zwischen dem 3'-Ende des ersten Oligonukleotids und der entsprechenden Region des zweiten oder dritten Oligonukleotids vorliegt.

7. Verfahren, wie in einem der vorhergehenden Ansprüche beansprucht, **dadurch gekennzeichnet, dass** die detektierbaren Elementtypen Fluorophor-Typen sind, die in dem ersten Oligonukleotid durch die Anwesenheit von mindestens einem Quencher nicht-detektierbar gemacht werden.

8. Verfahren, wie in einem der vorhergehenden Ansprüche beansprucht, **dadurch gekennzeichnet, dass** das detektierte modifizierte Einzelnukleosidtriphosphat entweder eine methylierte Cytosin- oder eine methylierte Adenin-Nukleotidbase enthält.

9. Verfahren, wie in einem der vorhergehenden Ansprüche beansprucht, **dadurch gekennzeichnet, dass** die Schritte (2a) und (3) bei verschiedenen Temperaturen durchgeführt werden.

10. Verfahren, wie in einem der vorhergehenden Ansprüche beansprucht, **dadurch gekennzeichnet, dass** das Sondensystem eine Vielzahl von ersten Oligonukleotidtypen umfasst, die jeweils mit einer anderen Einfangregion und charakteristischen ersten oder ersten und zweiten detektierbaren Elementtypen versehen sind.

11. Verfahren, wie in Anspruch 10 beansprucht, **dadurch gekennzeichnet, dass** bis zu vier unterschiedliche Sätze von Oligonukleotidsonden-Systemen verwendet werden, wobei das erste Oligonukleotid jedes Satzes eine Einfangregion, die für eine der charakteristischen Nukleotidbasen von natürlich vorkommender DNA oder RNA selektiv ist, und unterschiedliche erste oder Paare von ersten und zweiten detektierbaren Elementtypen aufweist.

12. Verfahren, wie in einem der vorhergehenden Ansprüche beansprucht, **dadurch gekennzeichnet, dass** der Schritt (1) ferner das Halten jedes Einzelnukleosidtriphosphats in einem entsprechenden Mikrotröpfchen umfasst, und dass die Schritte (2) bis (6) und (2a) in jedem Mikrotröpfchen ausgeführt werden.

13. Verfahren, wie in Anspruch 12 beansprucht, **dadurch gekennzeichnet, dass** die durch Anwenden von Schritt (6) auf jedes Mikrotröpfchen erhaltenen Ergebnisse zu einem Datenstrom zusammengefügt werden, der für die Sequenz der Nukleinsäure charakteristisch ist.

14. Biologisches Mehrkomponenten-Sondensystem, **dadurch gekennzeichnet, dass** es umfasst: (a) ein erstes einzelsträngiges Oligonukleotid, das mit ersten und zweiten Regionen von unterschiedlichen detektierbaren Elementtypen in einem nicht detektierbaren Zustand markiert ist, die sich jeweils auf der X'- und Y'-Seite einer dritten Region befinden, umfassend eine Einzelnukleotid-Einfangstelle, die komplementär zu einer der Nukleobasen von DNA oder RNA ist, aufweisend (1) eine assoziierte Restriktionsenzym-Erkennungsstelle und (2) eine Exonuklease-Blockierungsstelle auf ihrer X'-Seite, und (b) zweite und dritte nicht-markierte einzelsträngige Oligonukleotide, die befähigt sind zur jeweiligen Hybridisierung an die komplementären X'- und Y'-Seiten-Regionen auf dem ersten Oligonukleotid, welche die Einfangstelle flankieren, wobei entweder X' 3' liegt und Y' 5' liegt oder X' 5' liegt und Y' 3' liegt.

15. Biologisches Sondensystem, wie in Anspruch 14 beansprucht, **dadurch gekennzeichnet, dass** das zweite oder dritte Oligonukleotid eine Endonukleolyse-dirigierende Verknüpfung einschließt, die sich an oder nahe seinem X'- bzw. Y'-Ende befindet, so dass diese Verknüpfung einen Teil einer Restriktionsenzym-Erkennungsstelle umfasst, die in der verwendeten Sonde erzeugt wird, so dass nur der erste Oligonukleotidstrang der verwendeten Sonde befähigt ist, durch die genannte Restriktionsendonuklease gespalten zu werden.

16. Biologisches Sondensystem, wie in Anspruch 14 oder 15 beansprucht, **dadurch gekennzeichnet, dass** das Y'-Ende des zweiten Oligonukleotids und das X'-Ende des dritten Oligonukleotids durch eine Linkerregion verbunden sind.

17. Biologisches Sondensystem, wie in Anspruch 16 beansprucht, **dadurch gekennzeichnet, dass** die Linkerregion eine Oligonukleotidregion umfasst.

18. Biologisches Sondensystem, wie in einem der der Ansprüche 14 bis 17 beansprucht, **dadurch gekennzeichnet, dass** die detektierbaren Elemente in den ersten und zweiten Regionen Fluorophore sind, die gegebenenfalls mit Quenchern assoziiert sind.

19. Biologisches Sondensystem, wie in einem der der Ansprüche 14 bis 18 beansprucht, **dadurch gekennzeichnet, dass** das zweite Oligonukleotid länger ist als die X'-Seite-flankierende Region des ersten Oligonukleotids.

20. Biologisches Sondensystem, wie in einem der der Ansprüche 14 bis 19 beansprucht, **dadurch gekennzeichnet, dass** das Nukleotid am 3'-Ende des ersten Oligonukleotids eine Fehlpaarung mit dem entsprechenden Nukleotid im zweiten oder dritten Oligonukleotid ist.

21. Biologisches Sondensystem, wie in den Ansprüchen 14 bis 20 beansprucht, **dadurch gekennzeichnet, dass** das X'-Ende des dritten Oligonukleotids eine Region umfasst, die gegen Exonukleolyse resistent ist.

22. Biologisches Sondensystem, wie in den Ansprüchen 15 bis 21 beansprucht, **dadurch gekennzeichnet, dass** die Endonukleolyse -dirigierende Verknüpfung eine Phosphorothioat- oder Phosphoramidit-Verknüpfung ist.

23. Biologisches Sondensystem, wie in den Ansprüchen 14 bis 21 beansprucht, **dadurch gekennzeichnet, dass** es ein bis vier unterschiedliche erste Oligonukleotidtypen umfasst, die sich in der Sequenz, der für die Einfangregion charakteristischen Nukleotidbase und den verwendeten detektierbaren Elementen unterscheiden.

## Revendications

1. Procédé de séquençage d'un acide nucléique **caractérisé par** les étapes de (1) génération d'un courant de nucléotides simples par pyrophosphorolyse progressive de l'acide nucléique ; (2) production d'au moins une sonde à oligonucléotide utilisé substantiellement en double brin par une mise en réaction, en présence d'une polymérase et d'une ligase, de l'un des nucléotides simples avec un système de sonde correspondant comprenant (a) un premier oligonucléotide en simple brin, incluant une première et une deuxième régions respectivement marquées avec différents types d'éléments détectables caractéristiques, dans un état indétectable, situés respectivement sur les côtés X' et Y' d'une troisième région comprenant un élément d'un site de reconnaissance d'une enzyme de restriction, incluant un site de capture complémentaire au nucléotide simple et un site de blocage d'une exonucléase sur le côté X' de celui-ci, dans lequel soit X' est 3' et Y' est 5' soit X' est 5' et Y' est 3', et (b) un deuxième et un troisième oligonucléotides en simples brins étant capables de s'hybrider avec des régions complémentaires sur le premier oligonucléotide flanquant le site de capture ; (2a) soit en (i) traitant la sonde utilisée avec une endonucléase de restriction dépendante d'une modification pour couper seulement le brin du premier oligonucléotide au niveau du site de reconnaissance si et seulement si le nucléotide simple capturé comprend une nucléobase qui est structurellement modifiée soit en (ii) traitant la sonde utilisée avec une endonucléase de restriction sensible à une modification pour couper seulement le brin du premier oligonucléotide au niveau du site de reconnaissance si et seulement si le nucléotide simple capturé comprend une nucléobase qui n'est pas modifiée ; (3) digestion du brin du premier oligonucléotide de la sonde utilisée avec une enzyme ayant une activité exonucléolytique double brin dans la direction X'-Y' correspondant au premier oligonucléotide pour produire des éléments détectables dérivés soit de la première région, soit de la deuxième région ou bien des première et deuxième régions, dans un état détectable, et un quatrième oligonucléotide en simple brin qui est, au moins en partie, le complément de la séquence du premier oligonucléotide ; (4) mise en réaction du quatrième oligonucléotide avec un autre premier oligonucléotide pour produire un produit d'oligonucléotides substantiellement en double brin correspondant à la sonde utilisée ; (5) répétition des étapes (2a), (3) et (4) dans un cycle et (6) détection des éléments détectables libérés dans chaque itération de l'étape (3), dans lequel, si l'endonucléase de restriction employée n'est pas du type coupure, le deuxième ou le troisième oligonucléotide inclut une liaison dirigeant une endonucléolyse située au niveau de son extrémité X' ou Y' respectivement, ou étant proche de celle-ci, de sorte que cette liaison comprenne une partie d'un site de reconnaissance d'une enzyme de restriction créé dans la sonde utilisée telle que seul le brin du premier oligonucléotide de la sonde utilisée soit capable d'être coupé par ladite endonucléase de restriction.

2. Procédé, tel que revendiqué dans la revendication 1, **caractérisé en ce que** l'extrémité Y' du deuxième oligonucléotide et l'extrémité X' du troisième oligonucléotide sont reliées par une région lieuse.

3. Procédé, tel que revendiqué dans la revendication 2, **caractérisé en ce que** la région lieuse comprend une région d'oligonucléotide.

4. Procédé, tel que revendiqué dans les revendications 2 ou 3, **caractérisé en ce que** le quatrième oligonucléotide généré comprend une boucle fermée.

5. Procédé, tel que revendiqué dans l'une quelconque des revendications précédentes, **caractérisé en ce que** le deuxième oligonucléotide (a) s'hybride avec la région flanquante sur le côté X' de la région de capture et (b) est plus long que la région sur ce côté-là.

6. Procédé, tel que revendiqué dans l'une quelconque des revendications précédentes, **caractérisé en ce qu'**il y a au moins un mésappariement de bases nucléotidiques entre l'extrémité 3' du premier oligonucléotide et la région correspondante du deuxième ou du troisième oligonucléotide.

7. Procédé, tel que revendiqué dans l'une quelconque des revendications précédentes, **caractérisé en ce que** les types d'éléments détectables sont des types de fluorophores rendus indétectables dans le premier oligonucléotide par la présence d'au moins un extincteur.

8. Procédé, tel que revendiqué dans l'une quelconque des revendications précédentes, **caractérisé en ce que** le nucléoside simple triphosphate modifié détecté inclut une base nucléotidique soit de cytosine méthylée soit d'adénine méthylée.

9. Procédé, tel que revendiqué dans l'une quelconque des revendications précédentes, **caractérisé en ce que** les étapes (2a) et (3) sont effectuées à différentes températures.

10. Procédé, tel que revendiqué dans l'une quelconque des revendications précédentes, **caractérisé en ce que** le système de sonde comprend une pluralité de types de premiers oligonucléotides, chacun étant muni d'une région de capture différente, et de types premiers ou premier et deuxième d'éléments détectables caractéristiques.

11. Procédé, tel que revendiqué dans la revendication 10, **caractérisé en ce que** jusqu'à quatre ensembles différents de systèmes de sondes d'oligonucléotides sont employés, le premier oligonucléotide de chaque ensemble ayant une région de capture sélective pour l'une des bases nucléotidiques caractéristiques d'un ADN ou ARN existant naturellement ainsi que différents premiers ou paires de premiers et deuxièmes types d'éléments détectables.

12. Procédé, tel que revendiqué dans l'une quelconque des revendications précédentes, **caractérisé en ce que** l'étape (1) comprend en outre le fait de contenir chaque nucléoside simple triphosphate dans une microgoutte correspondante et que les étapes (2) à (6) et (2a) sont effectuées dans chaque microgoutte.

13. Procédé, tel que revendiqué dans la revendication 12, **caractérisé en ce que** les résultats obtenus en appliquant l'étape (6) à chaque microgoutte sont assemblés en un flux de données caractéristique de la séquence de l'acide nucléique.

14. Système de sonde biologique à plusieurs composants **caractérisé par** le fait de comprendre (a) un premier oligonucléotide en simple brin marqué avec une première et une deuxième régions de différents types d'éléments détectables, dans un état indétectable, situés respectivement sur les côtés terminaux X' et Y' d'une troisième région comprenant un site de capture d'un nucléotide simple complémentaire à l'une des nucléobases d'ADN ou d'ARN ayant (1) un site associé de reconnaissance d'une enzyme de restriction et (2) un site de blocage d'une exonucléase sur son côté X' et (b) un deuxième et un troisième oligonucléotides en simple brin non marqués étant capables de s'hybrider respectivement avec les régions des côtés X' et Y' complémentaires sur le premier oligonucléotide flanquant le site de capture, dans lequel soit X' est 3' et Y' est 5' soit X' est 5' et Y' est 3'.

15. Système de sonde biologique, tel que revendiqué dans la revendication 14, **caractérisé en ce que** le deuxième ou le troisième oligonucléotide inclut une liaison dirigeant une endonucléolyse située au niveau de son extrémité X' ou Y' respectivement, ou étant proche de celle-ci, de sorte que cette liaison comprenne une partie d'un site de reconnaissance d'une enzyme de restriction créé dans la sonde utilisée telle que seul le brin du premier oligonucléotide de la sonde utilisée soit capable d'être coupé par ladite endonucléase de restriction.

16. Système de sonde biologique, tel que revendiqué dans les revendications 14 ou 15, **caractérisé en ce que** l'extrémité Y' du deuxième oligonucléotide et l'extrémité X' du troisième oligonucléotide sont reliées par une région lieuse.

17. Système de sonde biologique, tel que revendiqué dans la revendication 16, **caractérisé en ce que** la région lieuse comprend une région d'oligonucléotide.

18. Système de sonde biologique tel que revendiqué dans l'une quelconque des revendications 14 à 17, **caractérisé en ce que** les éléments détectables dans les première et deuxième régions sont des fluorophores, en option associés à des extincteurs.

19. Système de sonde biologique, tel que revendiqué dans l'une quelconque des revendications 14 à 18, **caractérisé en ce que** le deuxième oligonucléotide est plus long que la région flanquant le côté X' du premier oligonucléotide.

20. Système de sonde biologique, tel que revendiqué dans l'une quelconque des revendications 14 à 19, **caractérisé en ce que** le nucléotide à l'extrémité 3' du premier oligonucléotide est un mésappariement avec le nucléotide correspondant dans le deuxième ou le troisième oligonucléotide.

21. Système de sonde biologique, tel que revendiqué dans l'une quelconque des revendications 14 à 20, **caractérisé en ce que** l'extrémité X' du troisième oligonucléotide comprend une région résistant à une exonucléolyse.

22. Système de sonde biologique, tel que revendiqué dans l'une quelconque des revendications 15 à 21, **caractérisé en ce que** la liaison dirigeant une endonucléolyse est une liaison de phosphorothioate ou de phosphoramidite.

23. Système de sonde biologique, tel que revendiqué dans l'une quelconque des revendications 14 à 21, **caractérisé par** le fait de comprendre d'un à quatre type(s) différent(s) de premiers oligonucléotides différant de par la séquence, la base nucléotidique caractéristique de la région de capture et les éléments détectables utilisés.


### SEQUENCE LISTING

<110> Base4 Innovation
<120> SINGLE NUCLEOTIDE DETECTION METHOD
<130> P103199EP
<160> 3
<170> PatentIn version 3.5
<210> 1
   <211> 50
   <212> DNA
   <213> Artificial sequence
<220>
   <223> Oligonucleotide 1
<220>
   <221> misc_feature
   <222> (17)..(18)
   <223> deoxythymidine nucleotide labelled with Atto 532 dye
<220>
   <221> misc_feature
   <222> (19)..(19)
   <223> deoxythymidine nucleotide labelled with a BHQ-1 quencher
<220>
   <221> misc_feature
   <222> (28)..(28)
   <223> 5-methylcytosine nucleotide
<220>
   <221> misc_feature
   <222> (35)..(36)
   <223> C3-spacer modification
<220>
   <221> misc_feature
   <222> (39)..(40)
   <223> deoxythymidine nucleotide (T) labelled with Atto 655 dye
<220>
   <221> misc_feature
   <222> (41)..(41)
   <223> deoxythymidine nucleotide labelled with a BHQ-2 quencher
<220>
   <221> misc_feature
   <222> (50)..(50)
   <223> hexanediol modification
<400> 1
   acatcacgac tatctatttc aggacggccc gttatcattt aaccgcacga 50
<210> 2
   <211> 32
   <212> DNA
   <213> Artificial sequence
<220>
   <223> Oligonucleotide 2
<220>
   <221> misc_feature
   <222> (28).. (29)
   <223> phosphorothioate linkage
<400> 2
   gtgatgttaa cgtgcggtta aatgataacg gg 32
<210> 3
   <211> 40
   <212> DNA
   <213> Artificial sequence
<220>
   <223> Oligonucleotide 3
<220>
   <221> misc_feature
   <222> (1)..(1)
   <223> 5' phosphate group
<220>
   <221> misc_feature
   <222> (40)..(40)
   <223> 3' inverted deoxythymidine base
<400> 3
   cgtcctgaaa tagatagtcg tgatgtctag catgacatat 40